# EUROPÄISCHE PATENTSCHRIFT

(11) **EP 0 937 158 B1**
(45) Veröffentlichungstag und Bekanntmachung des Hinweises auf die Patenterteilung: **27.12.2000**
(21) Anmeldenummer: 97945831.2
(22) Anmeldetag: 15.10.1997
(51) Int. Cl.: C12Q 1/56, G01N 33/86

(54) **VERFAHREN ZUR BESTIMMUNG DER KATALYTISCHEN AKTIVITÄT VON FAKTOR IXa**
METHOD FOR DETERMINING THE CATALYTIC ACTIVITY OF FACTOR IXa
DISPOSITIF POUR DETERMINER L'ACTIVITE CATALYTIQUE DU FACTEUR IXa

(30) Priorität: 31.10.1996 EP 96117470
(43) Veröffentlichungstag der Anmeldung: 25.08.1999
(73) Patentinhaber: Roche Diagnostics GmbH, 68305 Mannheim (DE)
(72) Erfinder: STÜRZEBECHER, Jörg, D-99094 Erfurt (DE)
(74) Vertreter: Witte, Hubert, Dr.
(86) Internationale Anmeldenummer: EP9705660
(87) Internationale Veröffentlichungsnummer: WO9818957

(56) Entgegenhaltungen:
- EP-A- 0 672 659
- WO-A-92/15324
- WO-A-97/47737
- US-A- 4 480 030
- US-A- 4 904 641
- US-A- 5 399 487

## Beschreibung

Gegenstand der Erfindung ist ein Verfahren zur Bestimmung der katalytischen Aktivität von Faktor IXa. Das erfindungsgemäße Verfahren ist geeignet zum Auffinden von Faktor IXa-Inhibitoren (Screening), zur Modulation der Blutgerinnung (therapeutische Anwendung) und zur Bestimmung von Faktor IX und Faktor IXa in Körperflüssigkeiten (diagnostische Anwendung).

Blutplasmaproteasen spielen sowohl eine Rolle bei der Blutgerinnung, dem Wundverschluß durch Fibrinbildung als auch bei der Fibrinolyse, der Clotauflösung. Nach einer Verletzung wird durch sequentielle Aktivierung (spezifische Proteolyse) von inaktiven Proenzymen zu aktiven Enzymen das "Verletzungssignal" amplifiziert, wodurch die Blutgerinnung eingeleitet und ein schneller Wundverschluß gewährleistet wird. Die Blutgerinnung kann über zwei Wege initiiert werden, dem intrinsischen Weg, bei dem alle Proteinkomponenten im Blut vorhanden sind, und dem extrinsischen Weg, bei dem ein Membranprotein, der sogenannte "tissue factor", eine kritische Rolle spielt.

Der molekulare Mechanismus der Bluthomöostase (Blutgerinnung, Fibrinolyse und die Regulation dieses Gleichgewichts) und der daran beteiligten Komponenten ist in einigen Übersichtsartikeln umfassend dargestellt worden (Furie, B. und Furie, B.C., Cell 53 (1988) 505-518; Davie, E.W. et al. Biochem. 30 (1991) 10363-10379; Bergmeyer, H.U. (ed.), Methods of Enzymatic Analysis, Vol. V, chapter 3, 3rd ed., Academic Press, New York (1983)).

Die Faktoren der Blutgerinnungskaskade sind sehr komplexe Proteine. Sie können in der Regel nur mit großem Aufwand in begrenzter Menge, mit schwankender Qualität, Homogenität und Reinheit aus der natürlichen Rohstoffquelle, dem Blutplasma, isoliert werden (Van Dam-Mieras, M.C.E, et al., In: Bergmeyer, H.U. (ed.), Methods of Enzymatic Analysis, Vol. V, 3rd ed., page 365-394, Academic Press, New York (1983)). Sie sind wesentlich an der Regulation der Bluthomöostase beteiligt, dem Gleichgewicht zwischen Blutgerinnung, Clot-bildung und Auflösung. Dieses gut regulierte System kann sowohl durch genetisch bedingte Defekte, wie z.B. bei der Hämophilie A (defekter Faktor VIII) und Hämophilie B (defekter Faktor IX), aus dem Gleichgewicht geraten. Akute Störungen können zu Herzinfarkt, Embolie und Gehirnschlag führen.

Es besteht deshalb ein Bedarf an Substanzen, mit denen das System der Blutgerinnung und Fibrinolyse je nach medizinischer Notwendigkeit beeinflußt werden kann. Zum Beispiel wird zur Behandlung der Hämophilie A und B aus Blut isolierter oder rekombinant hergestellter Faktor VIII bzw. Faktor IX verwendet. Zur Clotauflösung, z.B. nach Herzinfarkt, findet tPA ("tissue type plasminogen activator") und Streptokinase (bakterieller Plasminogenaktivator) Anwendung. Neben komplexen Proteinen werden auch Substanzen wie z.B. Hirudin (Peptid aus 65 Aminosäuren, Thrombininhibitor), Heparin (Heteroglykan, Cofaktor endogener Inhibitoren) und Vitamin-K Antagonisten (Inhibitoren der γ-Carboxylierung der Glu-Reste der GLA-Domäne) zur Hemmung der Blutgerinnung verwendet. Die verfügbaren Substanzen sind jedoch oft noch sehr teuer (Proteinfaktoren) und hinsichtlich ihrer medizinischen Anwendung nicht ideal (Nebenwirkungen), so daß ein Bedarf an Medikamenten besteht, mit dem die Blutgerinnung und Clotauflösung spezifischer moduliert werden kann.

Die Suche nach neuen Modulatoren (Aktivatoren, Inhibitoren) der Blutgerinnung, Fibrinolyse und Homöostase kann z.B. durch Screening von Substanzbibliotheken und anschließende Verbesserung einer identifizierten Leitstruktur durch "drug modelling" erfolgen. Dazu ist es notwendig, daß i) ein geeigneter Test und ii) das (die) Schlüsselprotein(e) [Target(s)] in ausreichender Menge und Qualität für ein Screening und für Kristallstrukturuntersuchungen (z.B. Verbesserung der Leitstruktur durch gezielte Vorhersage von Veränderungen anhand der 3D-Struktur aus Proteinkomponente und Leitstruktur) zur Verfügung stehen.

Faktor IXa (FIXa) ist ein interessantes Target für ein Inhibitorscreening zur Auffindung von Hemmstoffen zur Modulation der Blutgerinnung. Das bekannte Krankheitsbild der Hämophilie B (Faktor IXa-Defekt) berechtigt zur Annahme, daß spezifische Faktor IXa-Inhibitoren den bekannten Thrombininhibitoren hinsichtlich der doch beträchtlichen pleiotropen Nebenwirkungen überlegen sind.

Bisher scheiterte ein Screening auf FIXa-Inhibitoraktivität an der Verfügbarkeit und extrem niedrigen katalytischen Aktivität von FIXa.

Die Isolierung der inaktiven Serinprotease FIX (Zymogen) aus Blutplasma und die anschließende Aktivierung durch Proteolyse ist schwierig, aufwendig, teuer und liefert oft nicht die gewünschte Menge und Qualität. So beträgt die Plasmakonzentration für das inaktive Proteasezymogen FIX nur 0,5 mg/l (Furie, B. und Furie, B.C., Cell 53 (1988) 505-518). Zudem sind die aus dem Plasma isolierten und in vitro aktivierten Proteasepräparationen oft sehr heterogen und instabil.

Das inaktive FIX-Zymogen kann z. B. mittels gereinigtem FXIa aktiviert/konvertiert werden (Van Dam-Mieras, M.C.E.; Muller, A.D.; van Dieijen, G.; Hemker, H.C.: Blood coagulation factors II, V, VII, VIII, IX, X and XI: Determination with synthetic Substrates. In: Bergmeyer, H.U. (ed.): Methods of Enzymatic Analysis, Vol. V, Enzymes 3: Peptidases, Proteinases and Their Inhibitors, page 365-394, 3rd ed., Academic Press, New York (1983)).

Blutplasmaproteasen sind komplexe Glycoproteine, die zur Familie der Serinproteasen gehören. Sie werden in der Leber als inaktive Proenzyme (Zymogene) synthetisiert, ins Blut sezerniert und bei Bedarf durch spezifische Proteolyse, d.h. durch Spaltung von ein oder zwei Peptidbindungen, aktiviert. Sie sind strukturell hinsichtlich der Proteindomänenanordnung und Zusammensetzung sehr ähnlich (Furie, B. und Furie, B.C., Cell 53 (1988) 505-518).

Die Proteasen der Faktor IX-Familie (Faktor VII, IX, X, und Protein C) bestehen gemäß Furie, B. und Furie, B.C. aus
- einem Propeptid,
- einer GLA-Domäne,
- einer "aromatic amino acid stack" Domäne,
- zwei EGF-Domänen (EGF1 und EGF2),
- einer Zymogen Aktivierungsdomäne (Aktivierungspeptid, AP) und
- einer katalytischen Proteasedomäne (CD).

Zudem werden die Blutplasmaproteasen während der Sekretion posttranslational modifiziert:
- 11 - 12 Disulfidbrücken
- N- und/oder O-Glycosylierung (GLA-Domäne und Aktivierungspeptid)
   - Bharadwaj, D. et al., J. Biol. Chem. 270 (1995) 6537-6542
   - Medved, L.V. et al., J. Biol. Chem. 270 (1995) 13652-13659
- Abspaltung des Propeptides
- γ-Carboxylierung von Glu-Resten (GLA-Domäne)
- β-Hydroxylierung eines Asp-Restes (EGF-Domänen)
- Spaltung der Zymogenregion (teilweise).

Nach Aktivierung der Zymogene (zymogene Form des Proteins) durch spezifische Spaltung von ein oder zwei Peptidbindungen (Abspaltung eines Aktivierungspeptids) bestehen die enzymatisch aktiven Proteasen aus zwei Ketten, die entspechend ihrem Molekulargewicht mit schwerer und leichter Kette bezeichnet werden. Die beiden Ketten werden in der Faktor IX-Proteasefamilie durch eine intermolekulare Disulfidbrücke zwischen der EGF2-Domäne und der Proteasedomäne zusammengehalten. Die Zymogen-Enzym-Transformation (Aktivierung) führt zu Konformationsänderungen innerhalb der Proteasedomäne. Dadurch kann sich eine für die Proteaseaktivität notwendige essentielle Salzbrücke zwischen der N-terminalen Aminosäure der Proteasedomäne und einem Asp-Rest innerhalb der Proteasedomäne ausbilden. Für diese Untergruppe von Serinproteasen ist der N-terminale Bereich sehr kritisch und darf nicht modifiziert werden. Nur dann kann sich die für Serinproteasen typische "active site" mit der katalytischen Triade aus Ser, Asp und His ausbilden [Blow, D.M.: Acc. Chem. Res. 9 (1976) 145-152; Polgar, L.: In: Mechanisms of protease action. Boca Raton, Florida, CRC Press, chapter 3 (1989)].

Blutplasmaproteasen können klassisch durch Isolierung der inaktiven Zymogene aus dem Blut und anschließende Aktivierung oder rekombinant durch Expresssion der entsprechenden cDNA in einer geeigneten Säugetier-Zellinie oder Hefe hergestellt werden.

WO 92/15324 beschreibt die Herstellung und Reinigung von Faktor IX als Humansplasma sowie ein verbessertes Verfahren zur Verhinderung der katalytischen Spaltung von Faktor IX in Faktor IXa. Außerdem wird ein Verfahren zur Bestimmung von Faktor IX über die Bestimmung der partiellen Thromboplastinzeit (PTT) beschrieben.

In US-Patent 4,904,641 wird ein Verfahren zur Inaktivierung filtrierbarer Pathogene aus Blutplasmaprodukten beschrieben, wobei ein solches Produkt Faktor IX enthalten kann. Die Bestimmung von Faktor IX in solchen Präparationen erfolgt über die Bestimmung der aktivierten partiellen Thromboplastinzeit (PTT).

Im US-Patent 4,480,030 wird ein Verfahren zur quantitativen Bestimmung von Faktor Xa beschrieben, wobei als Substrat ein Tripeptid verwendet wird, welches kovalent einen abspaltbaren Chromophor gebunden enthält. Gemäß Tabelle 3 des US-Patents 4,904,641 wird nach der Bestimmung von Faktor IX die Kinetik der Virusinaktivierung in Gegenwart von Ethanoldampf bestimmt.

Die Herstellung von Gerinnungsfaktoren durch Expression / Sekretion der Zymogene bzw. aktiven Proteasen mittels eukaryontischer Wirts-/ Vektorsysteme wird beschrieben für **FVII**: Hagen, F.S. et al., EP 0 200 421; Pedersen, A.H. et al., Biochem. 28 (1989) 9391-9336; **FIX**: Lin, S.-W. et al., J. Biol. Chem. 265 (1990) 144-150; **FX:** Wolf, D.L. et al., J. Biol. Chem. 266 (1991) 13726-13730; **Protein C**: Bang, N.U. et al., EP 0 191 606.

In der Regel werden Wirtszellen verwendet, die in der Lage sind, die Gerinnungsfaktoren während des Sekretionsprozeßes entsprechend dem nativen Enzym posttranslational zu modifizieren. Die Zymogen-Enzym-Transformation erfolgt dann nachträglich während der "down stream"-Prozessierung, wie z.B. im Falle von Prothrombin oder Faktor X mit einem Aktivator aus Schlangengift (Sheehan, J.P. et al., J. Biol. Chem. 268 (1993) 3639-3645; Fujikawa, K. et al., Biochem. 11 (1972) 4892-4898).

Zwecks Zymogen-Enzym-Aktivierung in vivo (bereits während der Sekretion) wurden die natürlichen Zymogenschnittstellen bzw. das gesamte Aktivierungspeptid gegen Proteaseschnittstellen ausgetauscht (mehrere benachbarte basische Aminosäuren), die von im Sekretionsweg der Wirtszelle natürlich vorkommenden spezifisch spaltenden Proteasen wie z.B. Kex2 (Hefe) oder PACE (Säugetier Zellinien) gespalten werden können (**FX**: Wolf, D.L. et al., J. Biol. Chem. 266 (1991) 13726-13730; **Prothrombin**: Holly, R.D. und Foster, D.C., WO 93/13208).

Auch die Herstellung von Varianten von Gerinnungsfaktoren (**FX**: Rezaie, A.R. et al., J. Biol. Chem. 268 (1993) 8176-8180; **FIX**: Zhong, D.G. et al., Proc. Natl. Acad. Sci. USA 91 (1994) 3574-3578), Mutanten (**FX:** Rezaie, A.R. et al., J. Biol. Chem. 269 (1994) 21495-21499; **Thrombin**: Yee, J. et al., J. Biol. Chem. 269 (1994) 17965-17970; **FVII**: Nicolaisen, E.M. et al., WO 88/10295) und Chimären z.B. aus **FIX** und **FX** (Lin, S.-W. et al., J. Biol. Chem. 265 (1990) 144-150; Hertzberg, M.S. et al., J. Biol. Chem. 267 (1992) 14759-14766) mittels eukaryontischer Wirts-/ Vektorsysteme ist bekannt.

Die Expression in eukaryontischen Säugetier-Zellinien ist jedoch aufwendig, limitierend hinsichtlich der Expressionsleistung und teuer. Außerdem können unerwünschte posttranslationale Modifikationen erfolgen.

Die Herstellung von Blutplasmaproteasen durch Expression in Prokaryonten und anschließende Naturierung des Expressionsprodukts ist beschrieben bei Thogersen, H.C. et al. (WO 94/18227). Danach erfolgt die Naturierung von FX Varianten mittels eines zyklischen Naturierungsprozesses, bei dem das inaktive FX Protein mittels eines Metallchelatkomplexes ("poly(His)-affinity handle") in einer Chromatographiesäule fixiert ist. Dazu wurde ein Fusionsprotein verwendet bestehend aus einer verkürzten FX Variante (EGF1, EGF2 und Proteasedomäne), einer zusätzlichen FXa Proteaseerkennungssequenz und einer aus 6 Histidin-Resten bestehenden Fixierungshilfe am C-Terminus der katalytischen Domäne.

Überraschenderweise wurde gefunden, daß die katalytische Aktivität von Faktor IXa gegenüber Substraten durch Alkohole stimuliert werden kann und dadurch ein sehr empfindlicher Faktor IXa-Test auf einfache Weise aufgebaut werden kann.

Gegenstand der Erfindung ist demnach ein Verfahren zur Bestimmung von Faktor IXa in einer Probenlösung, dadurch gekennzeichnet, daß man der Probenlösung ein bestimmbares Faktor IXa-Substrat und einen mit Wasser homogen mischbaren und eine Phase bildenden Alkohol zusetzt und die Spaltung des Faktor IXa-Substrats als Maß für die Faktor IXa-Aktivität bestimmt. Es hat sich überraschenderweise gezeigt, daß die katalytische Faktor IXa-Aktivität durch Alkohole bis mehr als 20fach erhöht werden kann. Dadurch ist es möglich, die Faktor IXa-Aktivität in Probenlösungen direkt zu bestimmen. Eine Bestimmung von Faktor IX in Probelösungen, vorzugsweise in Körperflüssigkeiten wie Plasma, kann z.B. nach Aktivierung des Faktors IX zu Faktor IXa mittels "Russels viper venom" bzw. der aus dem Schlangengift gewonnenen Protease (RVV-X-Protease) erfolgen.

Ein weiterer Gegenstand der Erfindung ist die Verwendung des erfindungsgemäßen Bestimmungsverfahrens für Faktor IXa zum Screening auf Substanzen, welche die Faktor IXa-Aktivität modulieren (inhibieren oder aktivieren). Demnach ist ein Gegenstand der Erfindung ein Verfahren zur Identifikation einer Substanz, welche die Aktivität von Faktor IXa moduliert, dadurch gekennzeichnet, daß
a) ein Faktor IXa-Substrat durch ein Polypeptid mit Faktor IXa-Aktivität in definierter Konzentration in Gegenwart eines Alkohols gespalten wird und die Geschwindigkeit der Spaltung des genannten Substrats als Maß für die Faktor IXa-Aktivität bestimmt wird,
b) die genannte Aktivität in Gegenwart einer Testsubstanz gemessen wird,
c) die Aktivität mit und ohne Testsubstanz verglichen wird und
d) die Differenz der Aktivität als Maß für die Aktivitätsmodulation durch die Testsubstanz verwendet wird.

Bevorzugt wird die Inhibition von Faktor IXa durch eine Testsubstanz geprüft.

Als Faktor IXa-Substrate sind beispielsweise Faktor Xa-Substrate geeignet, wie sie in der EP-B 0 034 122 beschrieben sind. Insbesondere sind Substrate vom Typ R-Xxx-Gly-Arg-pNA geeignet, wobei Xxx für eine hydrophobe D-Aminosäure und pNA exemplarisch für eine bestimmbare Abgangsgruppe stehen. R ist analog zur EP-B 0 034 122 definiert. Bevorzugt sind Substrate der allgemeinen Formel I aus der EP-B 0 034 122, in der R³ = R⁴ = H bedeuten.

Als Substrate bevorzugt sind dabei Tripeptide mit einer hydrophoben D-Aminosäure am N-Terminus, wobei vorzugsweise eine Cyclohexyl-substitutierte hydrophobe D-Aminosäure verwendet wird. Besonders bevorzugt sind hierbei Cyclohexylglycin und Cyclohexylalanin.

Weitere bevorzugte Substrate sind beispielsweise Substrate, die auch von Faktor Xa gespalten werden, wie z. B. Chromozym X (Boehringer Mannheim GmbH, Moc-D-Nle-Gly-Arg-pNA) oder Pefachrom tPA (Pentapharm Ltd., Basel, MeSO₂-D-HHT-Gly-Arg-pNA). Vorzugsweise werden handelsübliche, optisch bestimmbare (vorzugsweise chromogene oder fluorogene) Substrate verwendet. Bei letzteren handelt es sich um chromogene Peptide/Substrate mit einem optisch leicht bestimmbaren abspaltbaren Rest (bevorzugt ist der p-Nitroanilinrest). Vorzugsweise wird das erfindungsgemäße Verfahren in einer Pufferlösung durchgeführt. Als Puffersubstanzen können alle im pH-Bereich 7-10 wirksamen Puffer (vorzugsweise zwischen pH 7,5-9,0) verwendet werden. Bevorzugt werden Tris-Puffer, Triethanolaminpuffer und Tris-Imidazolpuffer.

Die FIXa-Aktivitätsbestimmung bzw. der Screening-Assay wird vorzugsweise bei 20-40°C, besonders bevorzugt bei Raumtemperatur durchgeführt und die Menge der abgespaltenen, optisch bestimmbaren Gruppe photometrisch/fluorometrisch bestimmt. Vorzugsweise wird die Extinktion bei 405 nm bestimmt. Die enzymatische Aktivität und die kinetischen Konstanten werden aus der linearen Anfangssteigung gemäß der Michaelis-Mentengleichung bestimmt.

Faktor IX bzw. das Verhältnis von Faktor IX zu Faktor IXa kann analog nach Aktivierung von Faktor IX zu Faktor IXa bestimmt werden. Zur Bestimmung von Faktor IX in Plasma wird zunächst Faktor IX zu Faktor IXa durch "Russels viper venom" (vorzugsweise 0,2 mg/ml) bzw. RVV-X-Protease (vorzugsweise 0,1 mg/ml) in Gegenwart von CaCl₂ (10 mmol/l) bei 20-40°C, vorzugsweise bei 37°C aktiviert. Nach erfolgter Aktivierung (vorzugsweise 15 min.) wird Ethylenglykol und das spaltbare Substrat in Konzentrationen von 20-40% bzw. 0,2-1 mmol/1 zugesetzt.

Für einen Screeningtest hat es sich vorteilhaft erwiesen, Faktor IXa in einer Konzentration von 0,02-0,5 µmol/l, vorzugsweise 0.05 µmol/l und das spaltbare Substrat in einer Konzentration von 0,2-1 mmol/l zuzusetzen bei einer Testsubstanzkonzentration im µmol/l-Bereich. Als Alkohol werden vorzugsweise Ethylenglykol, Ethanol oder Methanol verwendet. Die Konzentration des Alkohols liegt vorzugsweise im Bereich von 20-40%. Als Faktor IXa kann vorzugsweise nativer humaner Faktor IXa oder Faktor IXa aus Schwein oder Rind oder rekombinant hergestellter humaner Faktor IXa verwendet werden. Besonders bevorzugt wird ein verkürzter humaner rekombinanter Faktor IXa, wie er in der EP 96 109 288.9 beschrieben ist, verwendet.

Enzymatisch aktiver rekombinanter Faktor IXa kann durch Expression einer entsprechenden DNA in Prokaryonten, Naturierung des Expressionsprodukts und enzymatische Spaltung hergestellt werden, wenn er zusammengesetzt ist aus einer FIXa-Serinproteasedomäne (katalytische Domäne), N-terminal verbunden mit einer EGF-Domäne (EGF1 und/oder EGF2) und einer Zymogen-Aktivierungsdomäne.

Vorzugsweise wird ein nicht glycosylierter, enzymatisch aktiver Faktor IXa, bestehend aus den folgenden Domänen verwendet:
a) der katalytischen FIXa-Proteasedomäne, N-terminal verbunden mit
b) einer Zymogenaktivierungsdomäne, N-terminal verbunden mit
c) einer EGF1- und/oder EGF2-Domäne (vorzugsweise EGF2 oder EGF1 und EGF2).

Vorzugsweise ist zwischen der Zymogenaktivierungsdomäne und der EGF-Domäne (oder den EGF-Domänen) ein Spacer mit bis zu 50 Aminosäuren eingefügt. Bei der Spaltung der erfindungsgemäßen zymogenen einkettigen Form in der Zymogenaktivierungsdomäne wird ein aktives Protein in einer zweikettigen Form erhalten. In der zweikettigen Form sind beide Ketten durch eine intermolekulare Disulfidbrücke verbunden. Vorzugsweise bestehen die erfindungsgemäßen Proteine aus der EGF2-Domäne, dem Aktivierungspeptid und der katalytischen Domäne von Faktor IX. Derartige Faktor-IX-Muteine sind in der Europäischen Patentanmeldung Nr. 96 110 959.2 beschrieben.

Im erfindungsgemäßen Bestimmungsverfahren kann jedes Plasma eingesetzt werden, bevorzugt wird Citratplasma.

Das Verfahren kann bei neutralen oder schwach alkalischen pH-Werten durchgeführt werden, vorzugsweise im pH-Bereich zwischen 7,5 und 9,0. Als Puffer können die in diesem Bereich wirksamen physiologisch unbedenklichen Puffer verwendet werden. Ferner können die für die Gerinnungstests üblichen Stabilisatoren und Konservierungsmittel, wie Rinderserumalbumin, Merthiolat und dergleichen ebenfalls zugesetzt werden.

Zusätzlich kann im erfindungsgemäßen Verfahren und zum erfindungsgemäßen Reagenz noch ein oberflächenaktives Mittel, vorzugsweise ein nichtionisches oberflächenaktives Mittel, wie Tween 80® enthalten sein. Die Konzentration beträgt in diesem Fall vorzugsweise 0,01-1 Vol.-%.

Ein weiterer Gegenstand der Erfindung ist ein Reagenz zur Bestimmung von Faktor IXa, welches ein durch Faktor IXa spaltbares, optisch bestimmbares Substrat, Alkohol und Puffer im Bereich zwischen pH 7 und 10 enthält.

Die folgenden Beispiele, Publikationen, das Sequenzprotokoll und die Abbildungen erläutern die Erfindung, deren Schutzumfang sich aus den Patentansprüchen ergibt, weiter. Die beschriebenen Verfahren sind als Beispiele zu verstehen, die auch noch nach Modifikationen den Gegenstand der Erfindung beschreiben.
- **Fig. 1**: zeigt den Einfluß von Ethanol und Ethylenglykol auf die Aktivität von rekombinantem rFIXa (0,48 µmol/l) und nativem FIXa (0,14 µmol/l). pH 7,4; Substrat: MeSO₂-D-HHT-Gly-Arg-pNA (1,02 mmol/l) - Abbildung zu Tabelle 2.
- **Fig. 2a**: zeigt den Einfluß von Ethylenglykol auf die Aktivität von rFIXa. **S1** = MeSO₂-D-HHT-Gly-Arg-pNA; **S2** = MOC-D-Nle-Gly-Arg-pNA; **S3** = MeSO₂-D-CHG-Gly-Arg-pNA; **S4** = MeSO₂-D-CHA-Gly-Arg-pNA; **S5** = MeSO₂-D-CHA-Gly-Arg-AMC; rFIXa = 0,48 µmol/l; pH = 7,4 - Abbildung zu Tabelle 3.
- **Fig. 2b**: zeigt den Einfluß von Ethanol auf die Aktivität von rFIXa. **S1** = MeSO₂-D-HHT-Gly-Arg-pNA; **S2** = MOC-D-Nle-Gly-Arg-pNA; **S3** = MeSO₂-D-CHG-Gly-Arg-pNA; **S4** = MeSO₂-D-CHA-Gly-Arg-pNA; **S5** = MeSO₂-D-CHA-Gly-Arg-AMC; rFIXa = 0,48 µmol/1; pH = 7,4 - Abbildung zu Tabelle 3.
- **Fig. 3a**: zeigt den Einfluß von Ethanol auf die Aktivität von P-Kallikrein, FXIIa, FXIa, FIXa, FXa und Thrombin (pH 7,4; 1,02 mmol/1 MeSO₂-D-HHT-Gly-Arg-pNA) - Abbildung zu Tabelle 5.
- **Fig. 3b**: zeigt den Einfluß von Ethylenglykol auf die Aktivität von P-Kallikrein, FXIIa, FXIa, FIXa, FXa und Thrombin (pH 7,4; 1,02 mmol/l MeSO₂-D-HHT-Gly-Arg-pNA) - Abbildung zu Tabelle 6.

### Methoden

### Rekombinante DNA-Technik

Zur Manipulation von DNA wurden Standardmethoden benutzt, wie sie bei Sambrook, J. et al. (1989) In; Molecular cloning: A laboratory manual. Cold Spring Harbor Laboratory Press, Cold Spring Harbor, New York, beschrieben sind. Die verwendeten molekularbiologischen Reagenzien wurden nach den Angaben des Herstellers eingesetzt.

### Proteinbestimmung

Die Proteinkonzentration der FIX-Varianten wurde durch Bestimmung der optischen Dichte (OD) bei 280 nm unter Verwendung des anhand der Aminosäuresequenz errechneten molaren Extinktionskoeffizienten ermittelt.

### Expressionsvektor

Der Vektor zur Expression der FIX-Variante basiert auf dem Expressionsvektor pSAM-CORE für core-Streptavidin. Die Herstellung und Beschreibung des Plasmids pSAM-CORE ist in der WO 93/09144 beschrieben.

Das core-Streptavidin-Gen wurde durch das Gen der gewünschten Variante im pSAM-CORE Vektor ersetzt.

### Beispiel 1

### Klonierung der katalytischen Domäne des FIX-Gens (Plasmid: pFIX-CD)

Die FIX-cDNA von Bp-Position 690-1403, kodierend für die FIX-Proteasedomäne von Aminosäureposition 181-415 (cDNA-Sequenz- und Aminosäuresequenz-Numerierung entsprechend der Publikation von McGraw, R.A. et al. (Proc. Natl. Acad. Sci. USA 82 (1985) 2847-2851) wurde unter Verwendung der PCR-Primer N1 (SEQ ID NO: 1) und N2 (SEQ ID NO: 2) und einer kommerziell erhältlichen humanen Leber cDNA Genbank (Vektor: Lamba ZAP® II) der Firma Stratagene (La Jolla, CA, U.S.A.) als Template DNA amplifiziert. Mittels der PCR-Primer wurde am 5'-Ende der kodierenden Region eine singuläre NcoI-Schnittstelle und ein ATG-Startkodon und am 3'-Ende der kodierenden Region eine singuläre HindIII-Schnittstelle eingeführt.

Das ca. 730 Bp lange PCR-Produkt wurde mit den Restriktionsendonukleasen NcoI und HindIII verdaut und das ca. 720 Bp lange NcoI/HindIII-FIX-Fragment nach Reinigung durch Agarosegelelektrophorese in das ca. 2,55 kBp lange NcoI/HindIII-pSAM-CORE Vektorfragment ligiert (s. WO 93/09144). Das gewünschte Plasmid pFIX-CD wurde durch Restriktionskartierung identifiziert und die durch PCR isolierte FIX-cDNA-Sequenz durch DNA-Sequenzierung überprüft.

### Beispiel 2

### Konstruktion des FIX-Protease-Gens mit EGF2-Domäne, Aktivierungspeptid und katalytischer Domäne (Plasmid: pFIX-EGF2-AP-CD)

Die FIX-cDNA von Bp-Position 402-986, kodierend für die EGF2-Domäne, das Aktivierungspeptid und den N-terminalen Bereich der FIXa-Proteasedomäne von Aminosäureposition 85-278 (McGraw et al., Proc. Natl. Acad. Sci. USA 82 (1985) 2847-2851) wurde unter Verwendung der PCR-Primer N3 (SEQ ID NO: 3) und N4 (SEQ ID NO: 4) und einer kommerziell erhältlichen humanen Leber-cDNA-Genbank (Vektor: Lamba ZAP® II) der Firma Stratagene (La Jolla, CA, U.S.A.) als Template-DNA amplifiziert. Mittels des PCR-Primers N3 wurde am 5'-Ende der kodierenden Region ein ATG-Startkodon und eine singuläre NcoI-Schnittstelle eingeführt.

Das ca. 590 Bp lange PCR-Produkt wurde mit den Restriktionsendonukleasen NcoI und BsmI verdaut und das ca. 360 Bp lange NcoI/BsmI-FIX-EGF2-AP-Fragment nach Reinigung durch Agarosegelelektrophorese in das ca. 3,2 kBp lange NcoI/BsmI-pFIX-CD-Vektorfragment (Beispiel 1) ligiert. Das gewünschte Plasmid pFIX-EGF2-AP-CD wurde durch Restriktionskartierung identifiziert und die durch PCR isolierte FIX-cDNA-Sequenz durch DNA-Sequenzierung überprüft.

### Beispiel 3

### a) Expression des FIXa-Protease-Gens in E. coli

Zur Expression des FIX-Gens mit Aktivierungspeptid wurde der E.coli-K12-Stamm UT5600 (Grodberg, J. und Dunn, J.J., J. Bacteriol. 170 (1988) 1245-1253) mit in Beispiel 2 beschriebenem Expressionsplasmid pFIX-EGF2-AP-CD (Ampicillin-Resistenz) und dem lacI^{q}-Repressorplasmid pUBS520 (Kanamycin-Resistenz, Herstellung und Beschreibung siehe: Brinkmann, U. et al., Gene 85 (1989) 109-114) transformiert. Statt des Stammes UT5600 können auch andere, dem Fachmann bekannte und zugängliche E.coli-Stämme, wie z.B. HB101 und E.coli B verwendet werden.

Die mit dem Expressionsplasmid pFIX-EGF2-AP-CD transformierten UT5600/pUBS520-Zellen wurden in Schüttelkultur in DYT-Medium (1% (w/v) Hefeextrakt, 1% (w/v) Bacto Tryptone, Difco, und 0,5% NaCl) mit 50-100 mg/l Ampicillin und 50 mg/l Kanamycin bei 37°C bis zu einer optischen Dichte bei 550 nm (OD₅₅₀) von 0,6-0,9 angezogen und anschließend mit LPTG (1-5 mmol/l Endkonzentration) induziert. Nach einer Induktionsphase von 4 - 8 Stunden (Std.) bei 37°C wurden die Zellen durch Zentrifugation (Sorvall RC-5B Zentrifuge, GS3 Rotor, 6000 UPM, 15 min) geerntet, mit 50 mmol/l Tris-HCl Puffer, pH 7,2 gewaschen und bis zur Weiterverarbeitung bei -20°C gelagert. Die Zellausbeute aus einer 11 Schüttelkultur betrug 4-5 g (Naßgewicht).

### b) Expressionsanalyse

Die Expression der mit Plasmid pFIX-EGF2-AP-CD transformierten UT5600/pUBS520-Zellen wurde analysiert. Dazu wurden Zellpellets aus jeweils 1 ml abzentrifugiertem Anzuchtmedium in 0,25 ml 10 mmol/l Tris-HCl, pH 7,2 resuspendiert und die Zellen durch Ultraschallbehandlung (2 Pulse a 30 s mit 50% Intensität) mit einem Sonifier® Cell Disruptor B15 der Firma Branson (Heusenstamm, Deutschland) aufgeschlossen. Die unlöslichen Zellbestandteile wurden sedimentiert (Eppendorf 5415 Zentrifuge, 14000 UPM, 5 min) und der Überstand mit 1/5 Volumen (Vol) 5xSDS-Probenpuffer (IxSDS-Probenpuffer: 50 mmol/l Tris-HCl, pH 6,8, 1% SDS, 1% Mercaptoethanol, 10% Glycerin, 0.001% Bromphenolblau) versetzt. Die unlösliche Zelltrümmerfraktion (Pellet) wurde in 0,3 ml IxSDS-Probenpuffer mit 6 - 8 M Harnstoff resuspendiert, die Proben 5 min bei 95°C inkubiert und erneut zentrifugiert Danach wurden die Proteine durch SDS-Polyacrylamid Gelelektrophorese (PAGE) aufgetrennt (Laemmli, U.K., Nature 227 (1970) 680-685) und mit Coomassie Brilliant Blue R Farbstoff angefärbt.

Die in E.coli synthetisierte FIX-Variante war homogen und wurde ausschließlich in der unlöslichen Zelltrümmerfraktion gefunden ("inclusion bodies", IBs). Die Expressionshöhe betrug 10-50% bezogen auf das E.coli-Gesamtprotein.

### Beispiel 4

### Zellyse, Solubilisierung und Naturierung

### a) Zellyse und Präparation der "inclusion bodies" (IBs)

Das Zellpellet aus 3 l Schüttelkultur (ca. 15 g Naßgewicht) wurde in 75 ml 50 mmol/l Tris-HCl, pH 7,2, resuspendiert. Die Suspension wurde mit 0,25 mg/ml Lysozym versetzt und 30 min bei 0°C inkubiert. Nach Zugabe von 2 mmol/l MgCl₂ und 10 µg/ml DNase I (Boehringer Mannheim GmbH, Kat.-Nr. 104159) wurden die Zellen mechanisch mittels Hochdruckdispersion in einer French® Press der Firma SLM Amico (Urbana, IL, U.S.A.) aufgeschlossen. Anschließend wurde die DNA 30 min bei Raumtemperatur (RT) verdaut. Der Ansatz wurde mit 37,5 ml 50 mmol/l Tris-HCl pH 7,2, 60 mmol/l EDTA, 1,5 mol/l NaCl, 6% Triton X-100 versetzt, weitere 30 min bei RT inkubiert und in einer Sorvall RC-5B Zentrifuge (GSA Rotor, 12000 UPM, 15 min) zentrifugiert. Der Überstand wurde verworfen, das Pellet mit 100 ml 50 mmol/l Tris-HCl, pH 7,2, 20 mmol/l EDTA versetzt, 30 min unter Rühren bei 4°C inkubiert und erneut sedimentiert. Der letzte Waschschritt wurde wiederholt. Die gereinigten IBs (1,5-2,0 g Naßgewicht, 25-30% Trockenmasse, 100-150 mg Protease) wurden bis zur Weiterverarbeitung bei -20°C gelagert.

### b) Solubilisierung und Derivatisierung der IBs

Die gereinigten IBs wurden in einer Konzentration von 100 mg IB-Pellet (Naßgewicht)/ml entsprechend 5-10 mg/ml Protein in 6 mol/l Guanidinium-HCl, 100 mmol/l Tris-HCl, 20 mmol/l EDTA, 150 mmol/l GSSG und 15 mmol/l GSH, pH 8,0 unter Rühren bei RT in 1-3 Std. suspendiert. Anschließend wurde der pH auf pH 5,0 eingestellt und die unlöslichen Bestandteile durch Zentrifugation (Sorvall RC-5B Zentrifuge, SS34 Rotor, 16000 UPM, 10 min) abgetrennt. Der Überstand wurde gegen 100 Vol 4-6 mol/l Guanidinium-HCl pH 5,0 für 24 Std. bei 4°C dialysiert.

### c) Naturierung

Die Naturierung der in 6 mol/l Guanidinium-HCl solubilisierten und mit GSSG/GSH derivatisierten Proteine wurde bei 4°C durch wiederholte (z.B. 3-fache) Zugabe von jeweils 0,5 ml IB-Solubilisat/Derivat zu 50 ml 50 mmol/l Tris-HCl, 0,5 mol/l Arginin, 20 mmol/l CaCl₂, 1 mmol/lEDTA und 0,5 mmol/l Cystein (pH 8,5) im Zeitabstand von 24 Std. und anschließende Inkubation für 48 Std. bei 4°C bewirkt. Nach Abschluß der Naturierungsreaktion wurden unlösliche Bestandteile durch Filtration mit einem Filtrationsgerät der Firma Satorius (Göttingen, Deutschland) bestückt mit Tiefenfilter K 250 der Firma Seitz (Bad Kreuznach, Deutschland) abgetrennt.

### d) Konzentrierung und Dialyse der Naturierungsansätze

Der klare proteasehaltige Überstand wurde durch Cross-Flow-Filtration in einer Minisette (Membran Typ: Omega 10K) der Firma Filtron (Karlstein, Deutschland) 10-15-fach konzentriert und zur Entfernung von Guanidinium-HCl und Arginin gegen 100 Vol 20 mmol/1 Tris-HCl und 50 mmol/l NaCl, pH 7,2 für 24 Std. bei 4°C dialysiert. Präzipitiertes Protein wurde durch Zentrifugation abgetrennt (Sorvall RC-5B Zentrifuge, SS34 Rotor, 16.000 UPM, 20 min) und der klare Überstand mit einer Nalgene® Einmalfiltrationseinheit (Porendurchmesser: 0,2 µm) der Firma Nalge (Rochester, NY, U.S.A.) filtriert.

### Beispiel 5

### Reinigung der naturierten FIX-Variante

Die FIX-Variante aus dem Naturierungsansatz kann bei Bedarf mit chromatographischen Methoden, die dem Fachmann bekannt sind, weiter gereinigt werden.

### a) Reinigung der FIX-Variante durch Ionenaustauschchromatographie an Q-Sepharose-ff

Der konzentrierte und gegen 20 mmol/l Tris-HCl und 50 mmol/l NaCl, pH 8,0 dialysierte Naturierungsansatz wurde auf eine mit dem gleichen Puffer äquilibrierte Q-Sepharose-ff-Säule (1,5 x 11 cm, V = 20 ml; Beladungskapazität: 10 mg Protein / ml Gel) der Firma Pharmacia Biotech (Freiburg, Deutschland) aufgetragen (2 Säulenvolumen/Stunde, 2 SV/Std.) und so lange mit dem Äquilibrierungspuffer gewaschen, bis die Absorption des Eluats bei 280 nm den Leerwert des Puffers erreichte. Die Elution des gebundenen Materials erfolgte durch einen Gradienten von 50- 500 mmol/l NaCl in 20 mmol/l Tris-HCl, pH 8,0 (2 SV/Std.). Die FIX-Variante wurde bei einer NaCl-Konzentration von 100-150 mmol/l eluiert. Die FIX-haltigen Fraktionen wurden durch nicht-reduzierende und reduzierende SDS PAGE identifiziert und der Elutionspeak vereinigt.

### b) Endreinigung der FIX-Variante durch Ionenaustauschchromatographie an Heparinsepharose CL-6B

Die vereinigten, nach Chromatographie an Q-Sepharose-ff erhaltenen FIX-haltigen Fraktionen wurden direkt auf eine mit 20 mmol/l Tris-HCl und 200 mmol/l NaCl, pH 8,0 äquilibrierte Heparinsepharose CL-6B Säule (1,5 x 11 cm; V = 20 ml, Beladungskapazität: 1 mg Protein / ml Gel) der Firma Pharmacia Biotech (Freiburg, Deutschland) aufgetragen (2 SV/Std.). Danach wurde so lange mit dem Äquilibrierungspuffer gewaschen, bis die Absorption des Eluats bei 280 nm den Leerwert des Puffers erreichte. Die Elution des gebundenen Materials erfolgte durch einen Gradienten von 0,2-1,0 mol/l NaCl in 20 mmol/l Tris-HCl, pH 8,0 (2 SV/Std.). Die FIX-Varianten wurden bei einer NaCl-Konzentration von 500-600 mmol/l eluiert. Die FIX-haltigen Fraktionen wurden durch nicht-reduzierende und reduzierende SDS PAGE identifiziert, der Elutionspeak vereinigt und gegen 20 mmol/l Tris-HCl, 50-200 mmol/l NaCl, 5 mmol/l CaCl₂, pH 7,8 dialysiert.

### Beispiel 6

### Aktivierung und Reinigung der FIX-Variante

Die naturierte gereinigte FIX-Variante wurde mit gereinigter "Russels viper venom" (RVV-X)-Protease aktiviert. Die RVV-X-Protease wurde, wie in der Publikation von Esmon, C.T. (Prothrombin activation, doctoral dissertation, Washington University, St. Louis, M0 (1973)) beschrieben, aus dem kommerziell erhältlichen Schlangengift (Lyophilisat) der Firma Sigma Aldrich Chemie GmbH (Deisenhofen, Deutschland) durch Gelfiltration gefolgt von Ionenaustausch-Chromatographie an Q-Sepharose ff gereinigt.

### a) Aktivierung und Reinigung der naturierten FIX-Variante

Die FIX-Variante wurde in einer Konzentration von 0,5 bis 2,0 mg/ml und einem Protease/Substrat-Verhältnis von 1:10 bis 1:20 in 20 mmol/l Tris-HCl, 50 mmol/l NaCl, 10 mmol/l CaCl₂, pH 7,8 bei 25°C verdaut. Der zeitliche Verlauf der enzymatischen FIX Aktivierung wurde durch Aktivitätsbestimmung mit einem chromogenen Substrat (siehe Beispiel 13 a) bis zur Vollständigkeit des Verdaus (Plateau, maximale Aktivierung) verfolgt. Dazu wurden aus dem Reaktionsansatz über einen Zeitraum von bis zu 24 Std. Proben (10 bis 100 µl) im Abstand von 3-4 Std. entnommen und die generierte FIXa Aktivität bestimmt. Nach Erreichen des Aktivitätsplateaus wurde der Aktivierungsansatz durch Negativ-Chromatographie an Q-Sepharose-ff gereinigt.

RVV-X und nicht aktivierte FIX-Variante binden unter den vorgegebenen Bedingungen an Q-Sepharose-ff, die aktivierte FIXa-Variante jedoch nicht.

Der Aktivierungsansatz wurde auf eine mit 20 mmol/l Tris-HCl, 50 mmol/l NaCl, pH 7,8 äquilibrierte Q-Sepharose-ff-Säule (1,0 x 10 cm, V = 8 ml) der Firma Pharmacia Biotech (Freiburg, Deutschland) aufgetragen (2 SV/Std.) und die Säule mit Äquilibrierungspuffer unter Fraktionierung entwickelt. Die proteasehaltigen Fraktionen wurden durch nicht-reduzierende und reduzierende SDS PAGE und Aktivitätsbestimmung identifiziert.

### Beispiel 7

### Charakterisierung der gereinigten Proteasevarianten

### a) Aktivitätstest

Die Aktivität der FIXa-Varianten wurde mit dem chromogenen Substrat Chromozym X (Boehringer Mannheim GmbH, Mannheim, Deutschland, Kat.-Nr. 789763) bestimmt. 10-100 µl Probe wurden mit 190-100 µl 50 mmol/l Tris-HCl, 150 mmol/l NaCl, 5 mmol/l CaCl₂, 0,1% PEG 8000, pH 8,0, auf 200 µl aufgefüllt, mit 20 µl Chromozym X (0,5-40 mmol/l) versetzt und in einem ELISA-Reader bei einer Wellenlänge von 405 nm und RT gegen einen Reagenzienleerwert vermessen. Die Aktivität und die kinetischen Konstanten wurden aus der linearen Anfangssteigung gemäß der Michaelis-Menten-Gleichung bestimmt.

### b) SDS-PAGE

Sowohl die Oligomeren- und Aggregatbildung durch intermolekulare Disulfidbrückenbildung als auch die Homogenität und Reinheit der naturierten aktivierten und gereinigten FIXa-Proteasevariante wurde durch nicht-reduzierende (minus Mercaptoethanol) und reduzierende (plus Mercaptoethanol) SDS PAGE (Laemmli, U.K., Nature 227 (1970) 680-685) untersucht.

### Beispiel 8

### Faktor IXa Test

Der FIXa-Test wurde mit rekombinant hergestelltem nativem humanem FIXa als Probe und dem Peptidsubstrat MeSO₂-D-HHT-Gly-Arg-pNA (Pefachrom tPA, Pentapharm Ltd., Basel, Schweiz) bzw. anderen chromogenen/fluorogenen Substraten vom Typ R-D-Xxx-Gly-Arg-pNA (EP-B 0 034 122) in Tris-HCl-Puffer und den zu testenden Substanzen (Alkohol, Lösungsmittel und Inhibitoren) durchgeführt. Die Reaktion wurde durch Zugabe von FIXa gestartet.

### Testprinzip:

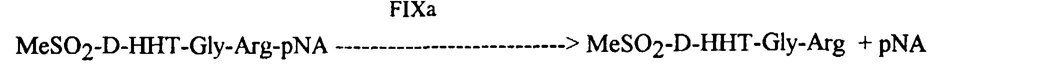

- Meßsignal:: pNA
- FIXa Substrate:: MOC-D-Nle-Gly-Arg-pNA (Chromozym X)
MeSO₂-D-HHT-Gly-Arg-pNA (Pefachrom tPA)
MeSO₂-D-CHG-Gly-Arg-pNA
MeSO₂-D-CHA-Gly-Arg-pNA
MeSO₂-D-CHA-Gly-Arg-AMC
- Abkürzungen:: pNA, p-Nitroanilin
AMC, 7-Amino-4-methyl-cumaryl
MOC, Methyloxycarbonyl
HHT, Hexahydrotyrosin
CHG, Cyclohexylglycin
CHA, Cyclohexylalanin

### Genereller Testansatz:

200 µl Puffer
- 50-100 mmol/l Tris-HCl, pH 7-9,5
- 100-200 mmol/l NaCl
- 5 mmol/l CaCl₂
- mit Zusätzen (Alkohole, Lösungsmittel und Inhibitoren)
   + 25 µl Peptidsubstrat(1-20 mmol/l)
   + 20 µl FIXa (0,02-0,5 µmol/l)

Der Testansatz wurde bei Raumtemperatur (25°C) in einer Mikrotiterplatte inkubiert und die Extinktionsänderung (ΔE/min) bei 405 nm mit einem ELISA-Reader bestimmt. Die Aktivität und die kinetischen Konstanten wurden aus der linearen Anfangssteigung gemäß der Michaelis-Menten-Gleichung bestimmt.

### Beispiel 9

### Einfluß von Alkoholen und organischen Lösungsmitteln auf die Aktivität von rFIXa

Untersucht wurde der Einfluß verschiedener, vollständig mit Wasser einphasig mischbarer Alkohole (Methanol, Ethanol, n-Propanol, i-Propanol, t-Butanol, Ethylenglykol und Glycerin) und organischer Lösungsmittel [Acetonitril, DMSO (Dimethylsulfoxid), und DMF (Dimethylformamid) sowie von gut löslichen mehrwertigen Alkoholen [m-Erythrit, PEG (Polyethylenglykol)] auf die Aktivität von rFIXa in Abhängigkeit von der Konzentration der Alkohole bzw. organischen Lösungsmittel (0-50%).

### Testansatz (Konzentration im Test):

- 41 mmol/l Tris-HCl, pH 7,4
- 82 mmol/l NaCl
- 4,1 mmol/ CaCl₂
- 1,02 mmol/l MeSO₂-D-HHT-Gly-Arg-pNA
- 0,48 µmol/l rFIXa (human)
- 0-50% (Alkohol bzw. organisches Lösungsmittel)

Die Ergebnisse sind in der Tabelle 1 dargestellt. Zur Verdeutlichung der Stimulation der katalytischen Aktivität von rFIXa wurde der Quotient aus V/V₀ gebildet.
V₀, Reaktionsgeschwindigkeit ohne Zusätze
V , Reaktionsgeschwindigkeit in Gegenwart von Zusätzen

**Tabelle 1**

| | **V/V**_{**0**} | | | | | | |
|---|---|---|---|---|---|---|---|
| **Zusatz [%]** | 0 | 8 | 16 | 25 | 33 | 41 | 50 |
| Methanol | 1 | 2,16 | 3,46 | 3,71 | 2,83 | 1,55 | 0,588 |
| Ethanol | 1 | 2,99 | 5,52 | 5,57 | 3,35 | 1,48 | 0,606 |
| n-Propanol | 1 | 3,43 | 2,26 | 0,339 | 0,186 | 0,056 | 0,056 |
| i-Propanol | 1 | 2,17 | 2,48 | 1,18 | 0,346 | 0,164 | 0,164 |
| t-Butanol | 1 | 2,05 | 1,67 | 0,702 | 0,368 | 0,263 | 0,263 |
| Ethylenglykol | 1 | 2,91 | 6,05 | 10,27 | 12,68 | 9,68 | 3,41 |
| Glycerin | 1 | 1,12 | 1,64 | 2,47 | 3,38 | 4,82 | 5,91 |
| m-Erythrit* | 1 | 1,16 | 1,35 | 1,49 | 1,56 | 1,64 | 1,77 |
| PEG* | 1 | 0,691 | 0,410 | 0,352 | 0,309 | 0,216 | 0,173 |
| Acetonitril | 1 | 0,828 | 0,314 | 0 | 0 | 0 | 0 |
| DMSO | 1 | 1,27 | 1,45 | 1,59 | 1,43 | 0,922 | 0,157 |
| DMF | 1 | 0,649 | 0,514 | 0 | 0 | 0 | 0 |

| | | | | | | | |
|---|---|---|---|---|---|---|---|
| *) g/100 ml | | | | | | | |

### Ergebnis:

Die Aktivität von rFIXa wird durch einige Alkohole (Ethylenglykol > Ethanol > Methanol) im Konzentrationsbereich von 15-40% ca. 5-15fach stimuliert. Glycerin stimuliert erst bei höheren Konzentrationen (25-50%), während der Einfluß von m-Erythrit gering ist. Dagegen hemmt Polyethylenglykol (PEG) die Aktivität von rFIXa. Bei Lösungsmitteln, die keine OH-Gruppe enthalten, hemmt nur Dimethylsulfoxid (DMSO) rFIXa bis 30% nicht, während Dimethylformamid (DMF) und Acetonitril das Enzym inaktivieren.

### Beispiel 10

### Einfluß von Ethanol und Ethylenglykol auf die Aktivität von rekombinantem humanem FIXa und nativem FIXa

Untersucht wurde, ob die beobachtete Stimulation von rFIXa (aktive rFXa-Variante bestehend aus EGFII-Domäne, prozessiertem Aktivierungspeptid und katalytischer Domäne) durch Ethanol und Ethylenglykol auch bei nativem FIXa (human) auftritt.

### Testansatz (Konzentration im Test):

- 41 mmol/1 Tris-HCl, pH 7,4
- 82 mmol/l NaCl
- 4,1 mmol/1 CaCl₂
- 1,02 mmol/l MeSO₂-D-HHT-Gly-Arg-pNA
- 0,48 µmol/l humaner rFIXa bzw.
- 0,14 µmol/l nativer FIXa (human)
- 0-50% Ethanol bzw. Ethylenglykol

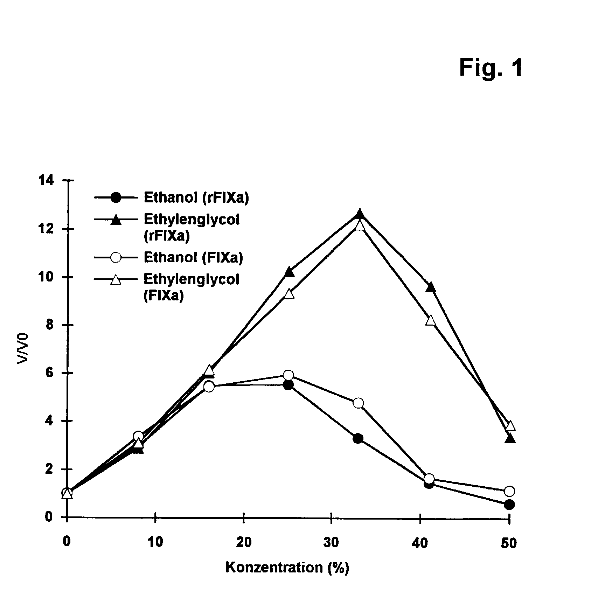

Die Ergebnisse sind in der Tabelle 2 zusammengestellt und in Figur 1 dargestellt.

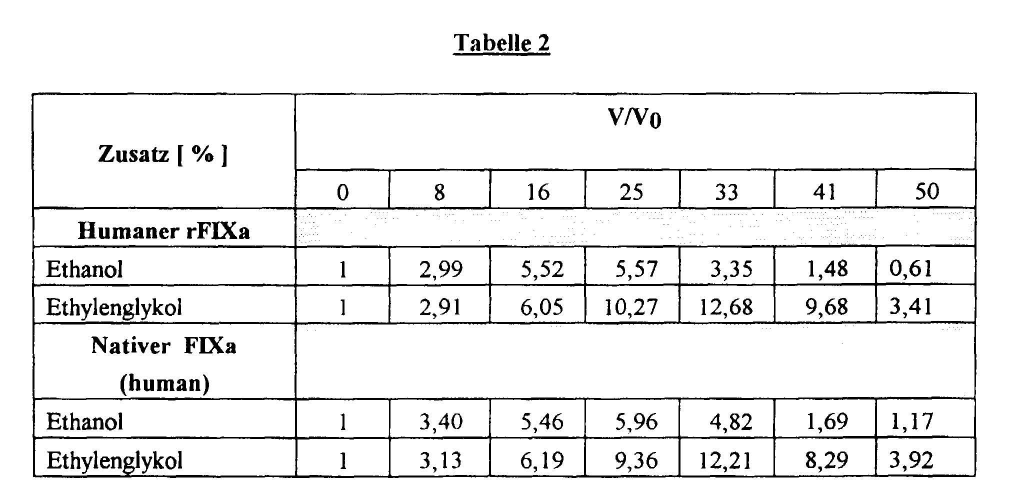

### Ergebnis:

Für rekombinanten humanen rFIXa und nativen humanen FIXa wurden keine Unterschiede hinsichtlich der Aktivierung durch Ethanol und Ethylenglykol gefunden.

### Beispiel 11

### Einfluß von Ethanol und Ethylenglykol auf die Aktivität von rFIXa gegenüber chromogenen und fluorogenen Peptidsubstraten

Untersucht wurde die Stimulation von humanem rFIXa durch Ethanol und Ethylenglykol unter Verwendung der Peptidsubstrate MeSO₂-D-HHT-Gly-Arg-pNA (Pefachrom tPA, Pentapharm Ltd., Basel, Schweiz), MOC-D-Nle-Gly-Arg-pNA (Chromozym X, Boehringer Mannheim GmbH, Mannheim, Deutschland, Kat.-Nr. 789763), MeSO₂-D-CHG-Gly-Arg-pNA (Pentapharm Ltd., Basel, Schweiz), MeSO₂-D-CHA-Gly-Arg-pNA (Pentapharm Ltd., Basel, Schweiz) und MeSO₂-D-CHA-Gly-Arg-AMC (Pentapharm Ltd., Basel, Schweiz).

### Testansatz chromogen (Konzentration im Test)

- 42 mmol/l Tris-HCl, pH 7,4
- 82 mmol/l NaCl
- 4,1 mmol/l CaCl₂
- 0-50% Ethylenglykol
- 1,02 mmol/l MeSO₂-D-HHT-Gly-Arg-pNA,
- 0,48 µmol/l rFIXa (human)
   bzw.
- 0,82 mmol/l MOC-D-Nle-Gly-Arg-pNA
- 0,48 µmol/l rFIXa (human)
   bzw.
- 1,02 mmol/l MeSO₂-D-CHG-Gly-Arg-pNA
- 0,28 µmol/l rFIXa (human) bzw.
- 1,02 mmol/1 MeSO₂-D-CHA-Gly-Arg-pNA
- 0,48 µmol/l rFIXa (human)

### Testansatz fluorogen (Konzentration im Test)

- 42 mmol/l Tris-HCl, pH 7,4
- 82 mmol/l NaCl
- 4,1 mmol/l CaCl₂
- 0-50% Ethylenglykol
- 0,51 mmol/l MeSO₂-D-CHA-Gly-Arg-AMC
- 0,08 µmol/l rFIXa (human)

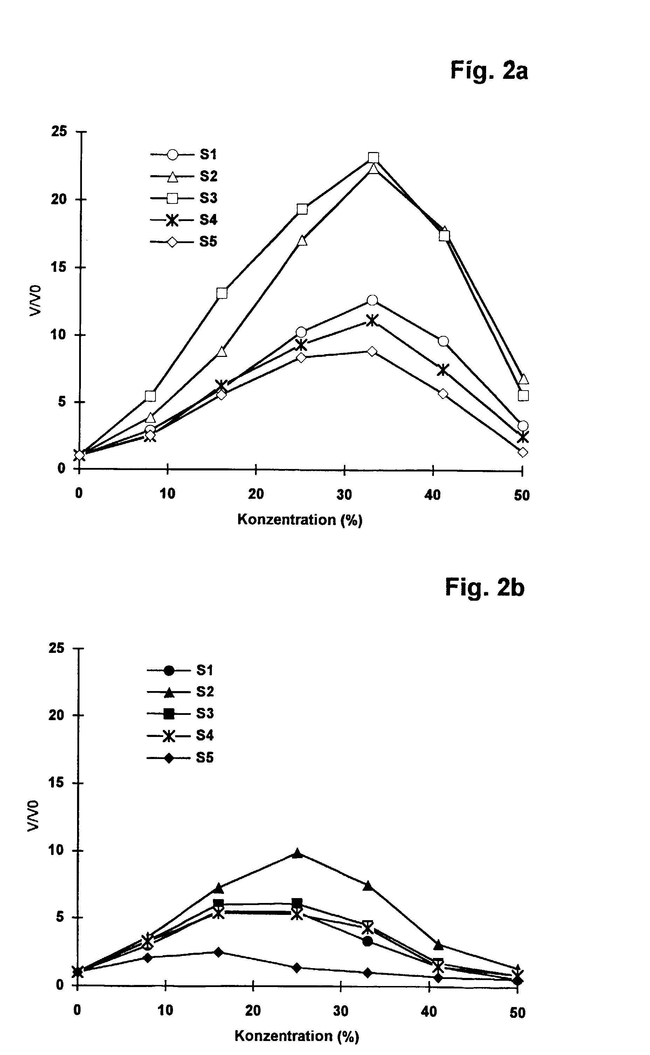

Die Ergebnisse sind in Tabelle 3 zusammengefaßt, die Befunde mit Ethylenglykol in Figur 2a und die Befunde mit Ethanol in Fig. 2b dargestellt.

**Tabelle 3**

| **Substrat** | **V/V**_{**0**} **Zusatz [%]** | | | | | | |
|---|---|---|---|---|---|---|---|
| **Zusatz** | 0 | 8 | 16 | 25 | 33 | 41 | 50 |
| MeSO₂-D-HHT-Gly-Arg-pNA | | | | | | | |
| Ethanol | 1 | 2,99 | 5,52 | 5,57 | 3,35 | 1,48 | 0,60 |
| Ethylenglykol | 1 | 2,91 | 6,05 | 10,27 | 12,68 | 9,68 | 3,41 |

| MOC-D-Nle-Gly-Arg-pNA | | | | | | | |
|---|---|---|---|---|---|---|---|
| Ethanol | 1 | 3,63 | 7,28 | 9,95 | 7,51 | 3,15 | 1,39 |
| Ethylenglykol | 1 | 3,91 | 8,85 | 17,07 | 22,42 | 17,80 | 6,96 |

| MeSO₂-D-CHG-Gly-Arg-pNA | | | | | | | |
|---|---|---|---|---|---|---|---|
| Ethanol | 1 | 3,33 | 6,02 | 6,16 | 4,50 | 1,74 | 0,87 |
| Ethylenglykol | 1 | 5,47 | 13,13 | 19,37 | 23,20 | 17,40 | 5,67 |

| MeSO₂-D-CHA-Gly-Arg-pNA | | | | | | | |
|---|---|---|---|---|---|---|---|
| Ethanol | 1 | 3,30 | 5,40 | 5,38 | 4,30 | 1,48 | 0,89 |
| Ethylenglykol | 1 | 2,84 | 6,26 | 9,34 | 11,18 | 7,54 | 2,56 |

| MeSO₂-D-CHA-Gly-Arg-AMC | | | | | | | |
|---|---|---|---|---|---|---|---|
| Ethanol | 1 | 2,09 | 2,50 | 1,37 | 1,01 | 0,70 | 0,58 |
| Ethylenglykol | 1 | 2,55 | 5,59 | 8,40 | 8,90 | 5,79 | 1,44 |

### Ergebnis:

Die Stimulierung von rFIXa durch Ethanol bzw. Ethylenglykol, ausgedrückt durch den Quotienten V/V₀, ist bei Verwendung der Peptidsubstrate MOC-D-Nle-Gly-Arg-pNA bzw. MeSO₂-D-CHG-Gly-Arg-pNA anstelle von MeSO₂-D-HHT-Gly-Arg-pNA weiter erhöht. Der Quotient V/V₀ ist oberhalb 25% Ethylenglykol um das 1,5-2fache größer als bei Substrat MeSO₂-D-HHT-Gly-Arg-pNA.

Auch die Spaltung des fluorogenen Substrats MeSO₂-D-CHA-Gly-Arg-AMC wird durch Alkohole in vergleichbarer Weise stimuliert. Die Spaltung der Tripeptide mit der Sequenz MeSO₂-D-CHA-Gly-Arg und C-terminalem AMC bzw. pNA wird durch Ethylenglykol (33%) auf das 9- bzw. 11 fache potenziert. Auf Grund der höheren Empfindlichkeit muß zur Spaltung des fluorogenen Substrats MeSO₂-D-CHA-Gly-Arg-AMC wesentlich (1/10) weniger rFIXa eingesetzt werden.

### Beispiel 12

### Einfluß von Ethylenglykol auf die Kinetik der Spaltung der chromogenen Peptidsubstrate MeSO₂-D-HHT-Gly-Arg-pNA, MOC-D-Nle-Gly-Arg-pNA und MeSO₂-D-CHG-Gly-Arg-pNA durch rekombinanten humanen FIXa und nativen FIXa

### Testansatz (Konzentration im Test)

- 42 mmol/l Tris-HCl, pH 7,4
- 82 mmol/l NaCl
- 4,1 mmol/l CaCl₂
- 0-50% Ethylenglykol
- MeSO₂-D-HHT-Gly-Arg-pNA (0,102 - 1,02 mmol/l)
- 0,48 µmol/l rFIXa (human)
   bzw.
- MeSO₂-D-HHT-Gly-Arg-pNA (0,102 - 1,02 mmol/l)
- 0,29 µmol/l nativer FIXa (human)
   bzw.
- MOC-D-Nle-Gly-Arg-pNA (0,102 - 1,02 mmol/l)
- 0,48 µmol/l rFIXa (human)
   bzw.
- MeSO₂-D-CHG-Gly-Arg-pNA (0,102 - 1,02 mmol/l)
- 0,28 µmol/1 rFIXa (human)

Die Bestimmung der kinetischen Konstanten (kₖₐₜ/Kₘ) erfolgte graphisch nach Lineweaver-Burk (Kₘ bzw. kₖₐₜ können nicht getrennt bestimmt werden, da die Geraden durch den Nullpunkt gehen).

**Tabelle 4a**

| **Substrat: MeSO**_{**2**}**-D-HHT-Gly-Arg-pNA** | | | | | | | |
|---|---|---|---|---|---|---|---|
| | **k**_{**kat**}**/K**_{**m**} **[1/mmol*s] Ethylenglykolkonzentration [%]** | | | | | | |
| **Enzym** | 0 | 8 | 16 | 25 | 33 | 41 | 50 |
| rFIXa | n.1. | 0,348 | 0,515 | 1,018 | 1,018 | 0,692 | 0,172 |
| nativer FIXa | n.1 | 0,299. | 0,737 | 1,169 | 1,395 | 0,922 | 0,390 |

**Tabelle 4b**

| **Verschiedene Substrate, rFIXa** | | | | | | | |
|---|---|---|---|---|---|---|---|
| | **k**_{**kat**}**/K**_{**m**} **[1/mmol*s] Ethylenglykolkonzentration [%]** | | | | | | |
| **Substrat** | 0 | 8 | 16 | 25 | 33 | 41 | 50 |
| MeSO₂-D-HHT-Gly-Arg-pNA | n.1. | 0,348 | 0,515 | 1,018 | 1,018 | 0,692 | 0,172 |
| MOC-D-Nle-Gly-Arg-pNA | n.1 | n.1. | 0,326 | 0,565 | 0,552 | 0,360 | 0,148 |
| MeSO₂-D-CHG-Gly-Arg-pNA | n.1. | 0,637 | 1,974 | 2,880 | 3,480 | 1,929 | 0,576 |
| n.l. = nicht linear | | | | | | | |

### Ergebnis:

Die Stimulierung der durch rekombinanten bzw. nativen humanen FIXa katalysierten Spaltung chromogener Peptidsubstrate wird auch für die kinetischen Konstanten gefunden, kₖₐₜ/kₘ steigt bis zu einer Ethylenglykol-Konzentration von 33% an. Für das Substrat MeSO₂-D-CHG-Gly-Arg-pNA wird die höchste katalytische Aktivität, d.h. der größte Wert von kₖₐₜ/Kₘ, bei 33% Ethylenglykol gefunden. Für die unbeeinflußten Reaktion ist die Auftragung nicht linear, es kann keine Konstante bestimmt werden.

### Beispiel 13

### Einfluß von Ethanol und Ethylenglykol auf die Aktivität von FIXa im Vergleich mit Plasmakallikrein , FXIIa, FXIa, FXa und Thrombin

### FIXa Testansatz (Konzentration im Test)

- 42 mmol/l Tris-HCl, pH 7,4
- 82 mmol/l NaCl
- 4,1 mmol/l CaCl₂
- 1,02 mmol/l MeSO₂-D-HHT-Gly-Arg-pNA
- 0,14 µmol/l nativer FIXa (human)
- 0-50% Ethanol bzw. Ethylenglykol

### Plasmakallikrein Testansatz (Konzentration im Test)

- 42 mmol/l Tris-HCl, pH 7,4
- 82 mmol/l NaCl
- 4,1 mmol/l CaCl₂
- 1,02 mmol/l MeSO₂-D-HHT-Gly-Arg-pNA
- 0,0176 U/ml Plasmakallikrein (human)
- 0-50% Ethanol bzw. Ethylenglykol

### FXIIa Testansatz (Konzentration im Test)

- 42 mmol/l Tris-HCl, pH 7,4
- 82 mmol/l NaCl
- 4,1 mmol/l CaCl₂
- 1,02 mmol/l MeSO₂-D-HHT-Gly-Arg-pNA
- 0,0045 U/ml FXIIa (human)
- 0-50% Ethanol bzw. Ethylenglykol

### FXIa Testansatz (Konzentration im Test)

- 42 mmol/l Tris-HCl, pH 7,4
- 82 mmol/l NaCl
- 4,1 mmol/l CaCl₂
- 1,02 mmol/l MeSO₂-D-HHT-Gly-Arg-pNA
- 0,0012 µmol/l FXIa (human)
- 0-50% Ethanol bzw. Ethylenglykol

### FXa Testansatz (Konzentration im Test)

- 42 mmol/l Tris-HCl, pH 7,4
- 82 mmol/l NaCl
- 4,1 mmol/l CaCl₂
- 1,02 mmol/l MeSO₂-D-HHT-Gly-Arg-pNA
- 0,088 µmol/l FXa (Rind)
- 0-50% Ethanol bzw. Ethylenglykol

### Thrombin Testansatz (Konzentration im Test)

- 42 mmol/l Tris-HCl, pH 7,4
- 82 mmol/l NaCl
- 4,1 mmol/l CaCl₂
- 1,02 mmol/l MeSO₂-D-HHT-Gly-Arg-pNA
- 0,045 µmol/l Thrombin (Rind)
- 0-50% Ethanol bzw. Ethylenglykol

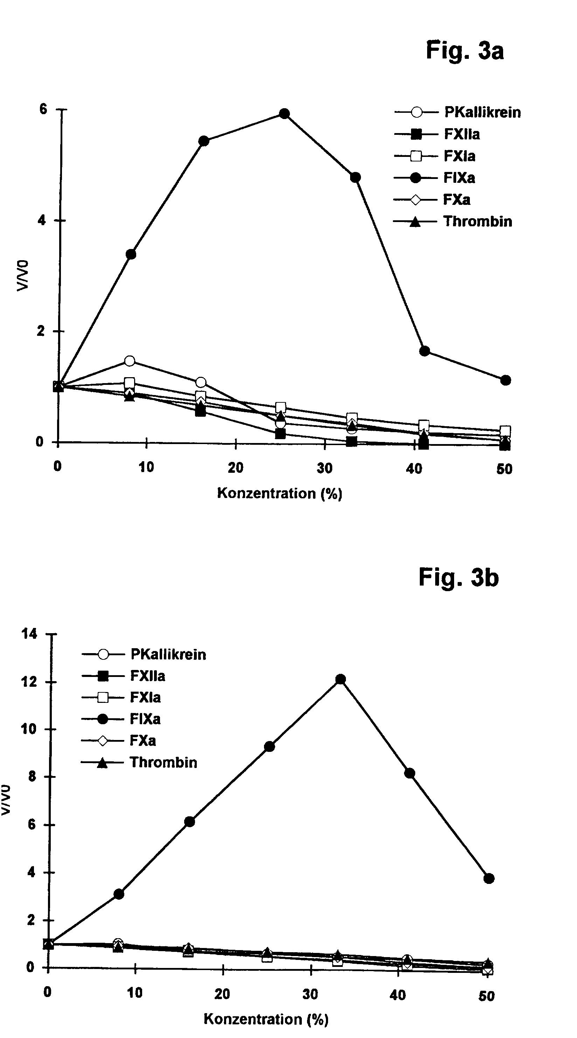

Die Ergebnisse sind in Tabelle 5 und 6 zusammengefaßt, die Beeinflussung durch Ethanol ist in Fig. 3a und die Beeinflussung durch Ethylenglykol in Figur 3b dargestellt.

**Tabelle 5**

| **Protease** | **V/V**_{**0**} **Ethanolkonzentration [%]** | | | | | | |
|---|---|---|---|---|---|---|---|
| | 0 | 8 | 16 | 25 | 33 | 41 | 50 |
| PKallikrein | 1 | 1,48 | 1,10 | 0,377 | 0,280 | 0,215 | 0,174 |
| FXIIa | 1 | 0,904 | 0,579 | 0,187 | 0,051 | 0,009 | 0 |
| FXIa | 1 | 1,08 | 0,845 | 0,657 | 0,469 | 0,353 | 0,258 |
| FIXa | 1 | 3,40 | 5,46 | 5,96 | 4,82 | 1,69 | 1,17 |
| FXa | 1 | 0,899 | 0,752 | 0,510 | 0,377 | 0,203 | 0,086 |
| Thrombin | 1 | 0,846 | 0,685 | 0,507 | 0,343 | 0,184 | 0,087 |

**Tabelle 6**

| **Protease** | **V/V**_{**0**} **Ethylenglykolkonzentration [%]** | | | | | | |
|---|---|---|---|---|---|---|---|
| | 0 | 8 | 16 | 25 | 33 | 41 | 50 |
| PKallikrein | 1 | 1,05 | 0,763 | 0,676 | 0,536 | 0,478 | 0,290 |
| F XIIa | 1 | 0,892 | 0,729 | 0,551 | 0,355 | 0,187 | 0,103 |
| FXIa | 1 | 0,922 | 0,790 | 0,569 | 0,422 | 0,275 | 0,165 |
| FIXa | 1 | 3,13 | 6,19 | 9,36 | 12,21 | 8,29 | 3,92 |
| FXa | 1 | 0,973 | 0,902 | 0,741 | 0,558 | 0,347 | 0,191 |
| Thrombin | 1 | 0,924 | 0,862 | 0,763 | 0,653 | 0,508 | 0,395 |

### Ergebnis:

Von den Gerinnungsfaktoren des intrinsischen Aktivierungsweges - Plasmakallikrein, FXIIa, FXIa, FIXa, FXa und Thrombin - wird nur FIXa durch Ethanol bzw. Ethylenglykol aktiviert. Eine geringfügige Aktivierung wird in Gegenwart von 10-20% Ethanol bei Plasmakallikrein beobachtet.

### Beispiel 14

### Einfluß des pH auf die Aktivität von rFIXa und nativem FIXa in Gegenwart von Ethylenglykol

### Testansatz (Konzentration im Test)

- 42 mmol/l Tris-HCl, pH 7,0 - 10,0
- 82 mmol/l NaCl
- 4,1 mmol/l CaCl₂
- 1,02 mmol/l MeSO₂-D-HHT-Gly-Arg-pNA, MOC-D-Nle-Gly-Arg-pNA bzw. MeSO₂-D-CHG-Gly-Arg-pNA
- 25% Ethylenglykol
- 0,28 µmol/l rFIXa (human)
   bzw.
- 0,14 µmol/l nativer FIXa (human)

Die Ergebnisse sind in Tabelle 7a-d dargestellt.

**Tabelle 7a**

| **Substrat: MeSO**_{**2**}**-D-HHT-Gly-Arg-pNA, rFIXa (human)** | | | | | | | | | |
|---|---|---|---|---|---|---|---|---|---|
| | **mE/min** | | | | | | | | |
| **Zusatz/pH** | 7,0 | 7,5 | 8,0 | 8,25 | 8,5 | 8,75 | 9,0 | 9,5 | 10,0 |
| ohne | 6,5 | 10,7 | 16,0 | 17,0 | 16,3 | 16,3 | 16,0 | 12,9 | 10,4 |
| Ethylenglykol [25%] | 56,5 | 124,5 | 173,6 | 176,0 | 182,1 | 170,7 | 164,1 | 112,0 | 110,1 |

**Tabelle 7b**

| **Substrat: MeSO**_{**2**}**-D-HHT-Gly-Arg-pNA, nativer FIXa (human)** | | | | | | | | | |
|---|---|---|---|---|---|---|---|---|---|
| | **mE/min** | | | | | | | | |
| **Zusatz/pH** | 7,0 | 7,5 | 8,0 | 8,25 | 8,5 | 8,75 | 9,0 | 9,5 | 10,0 |
| ohne | 3,3 | 6,8 | 10,0 | 10,5 | 10,3 | 9,9 | 9,3 | 7,4 | 6,2 |
| Ethylenglykol [25%] | 23,7 | 63,0 | 101,0 | 106,7 | 115,4 | 110,9 | 105,6 | 100,6 | 77,2 |

**Tabelle 7c**

| **Substrat: MOC-D-Nle-Gly-Arg-pNA, rFIXa (human)** | | | | | | | | | |
|---|---|---|---|---|---|---|---|---|---|
| | **mE/min** | | | | | | | | |
| **Zusatz/pH** | 7,0 | 7,5 | 8,0 | 8,25 | 8,5 | 8,75 | 9,0 | 9,5 | 10,0 |
| ohne | 3,1 | 5,2 | 6,8 | 7,3 | 7,9 | 7,1 | 7,1 | 7,0 | 5,2 |
| Ethylenglykol [25%] | 31,5 | 70,1 | 99,7 | 102,7 | 104,3 | 101,6 | 97,3 | 70,7 | 68,3 |

**Tabelle 7d**

| **Substrat: MeSO**_{**2**}**-D-CHG-Gly-Arg-pNA, rFIXa (human)** | | | | | | | | | |
|---|---|---|---|---|---|---|---|---|---|
| | **mE/min** | | | | | | | | |
| **Zusatz/pH** | 7,0 | 7,5 | 8,0 | 8,25 | 8,5 | 8,75 | 9,0 | 9,5 | 10,0 |
| ohne | 6,5 | 11,3 | 18,1 | 18,8 | 19,6 | 20,3 | 18,4 | 14,8 | 11,3 |
| Ethylenglykol [25%] | 154,7 | 289,8 | 372,4 | 382,2 | 389,8 | 395,6 | 373,3 | 255,6 | 244,5 |

### Ergebnis:

Die pH-Abhängigkeit der katalytischen Aktivität von rFIXa wird durch Alkohole, wie am Beispiel Ethylenglykol gezeigt, nicht verändert. So werden zwischen pH 7,0 und 10,0 in Gegenwart von 25% Ethylenglykol die Spaltungsraten im gleichen Verhältnis gesteigert. Dabei erhöhen sich die Spaltungsraten in Gegenwart von Substrat MeSO₂-D-HHT-Gly-Arg-pNA auf das 10fache, bei MOC-D-Nle-Gly-Arg-pNA auf das 14fache und bei MeSO₂-D-CHG-Gly-Arg-pNA auf das 20fache. Der optimale pH-Bereich liegt zwischen pH 8,25 und 8,75. Am effektivsten wird Substrat MeSO₂-D-CHG-Gly-Arg-pNA gespalten.

### Beispiel 15

### Test zur Auffindung von FIXa-Inhibitoren

Spezifische FIXa-Inhibitoren können durch Hemmung der FIXa-Aktivität identifiziert werden. Dazu wird die FIXa-Aktivität mit einem Substrat vom Typ R-D-Xxx-Gly-Arg-pNA (Xxx = hydrophobe Aminosäure) in Ab- und Anwesenheit der zu testenden Substanz oder eines Substanzgemisches bestimmt und durch Quotientenbildung die prozentuale Hemmung errechnet. Die Hemmkonstante Kᵢ wird aus einer Hemmkinetik ermittelt. Die Gegenwart hoher Konzentrationen bestimmter Alkohole (vorzugsweise Ethylenglykol) verstärkt das Meßsignal.

### Testprinzip:

- Meßsignal:: pNA
- FIXa-Substrate:: MeSO₂-D-HHT-Gly-Arg-pNA (Pefachrom tPA)
MOC-D-Nle-Gly-Arg-pNA (Chromozym X)
MeSO₂-D-CHG-Gly-Arg-pNA
- FIXa:: rekombinant hergestellter rFIXa (human)

### Testansatz:

200 µl Puffer (100 mmol/l Tris-HCl, 100 mmol/l NaCl, 5 mmol/l CaCl₂, 20-40% Alkohol, pH 7-9)
   der Puffer enthält den Inhibitor in unterschiedlicher Konzentration
+ 25 µl Peptidsubstrat
+ 20 µl rFIXa (human)

Der Testansatz wurde bei RT in einer Mikrotiterplatte inkubiert und die Extinktionsänderung (ΔE/min) bei 405 nm mit einem ELISA-Reader bestimmt. Die Reaktion kann auch nach 5-10 min durch Abstoppen mit Essigsäure (25 µl, 50%) beendet werden, die Extinktion wird bei 405 nm gegen einen Reagenzienleerwert bestimmt.

### Beispiel 16

### Einfluß bekannter Proteaseinhibitoren auf die Aktivität von rFIXa bei unterschiedlichem pH, Alkohol und Alkoholkonzentration

### Testansatz (Konzentration im Test)

- 42 mmol/l Tris-HCl, pH 7,4 bzw. 8,5
- 82 mmol/l NaCl
- 4,1 mmol/l CaCl₂
- Substrat MeSO₂-D-CHG-Gly-Arg-pNA (1,02 mmol/l)
- 0,028 - 0,57 µmol/l rFIXa (human)
- 4,1% Ethanol bzw. 33% Ethylenglykol
- Inhibitor in abgestuften Konzentrationen (4,1-163 µmol/l)

Als Hemmstoffe wurden bekannte synthetische Inhibitoren vom Typ der 3-Amidinophenylalanine eingesetzt (Stürzebecher et al., J. Enzym. Inhibition 9 (1995) 87 und WO 92/08709 (1991)). Beispielhaft wurde die Hemmwirkung bei einer niedrigen und einer hohen Alkoholkonzentration untersucht.

**Tabelle 8a**

| **Hemmung der Aktivität von Faktor IXa (in %) durch den Inhibitor Nα-(2,4,6- Triisopropylbenzolsulfonyl)-3-amidino-(D,L)-phenylalanin-isonipecotincarbonsäure [TIPPS-(3-Am)Phe-iNip-OH]** | | | | | | |
|---|---|---|---|---|---|---|
| | **Inhibitor-Konzentration [µmol/l]** | | | | | |
| **Bedingungen** | 4,1 | 8,2 | 16,3 | 40,8 | 81,6 | 163 |
| pH 7,4; 4,1% Ethanol 0,57 µmol/l rFIXa | 89,5 | 87,6 | 76,2 | 55,8 | 36,3 | 23,8 |
| pH 8,5; 4,1% Ethanol 0,28 µmol/l rFIXa | 90,6 | 85,2 | 74,8 | 55,2 | 35,3 | 20,8 |
| pH 7,4; 33% Ethylenglykol 0,057 µmol/l rFIXa | 86,9 | 79,4 | 61,7 | 38,9 | 21,2 | 15,0 |
| pH 8,5; 33% Ethylenglykol 0,028 µmol/l rFIXa | 85,6 | 72,8 | 58,0 | 33,6 | 20,7 | 15,8 |

**Tabelle 8b**

| **Hemmung der Aktivität von Faktor IXa (in %) durch den Inhibitor Nα-(2- Naphthylsulfonyl)-3-amidino-(D,L)-phenylalanin-1,2,3,4-tetrahydroisochinolin-3-carbonsäure [βNAPS-(3-Am)Phe-TIC-OH]** | | | | | | |
|---|---|---|---|---|---|---|
| | **Inhibitor-Konzentration [µmol/l]** | | | | | |
| **Bedingungen** | 4,1 | 8,2 | 16,3 | 40,8 | 81,6 | 163 |
| pH 7,4; 4,1% Ethanol 0,57 µmol/l rFIXa | 97,2 | 97,2 | 88,2 | 75,4 | 57,0 | 25,7 |
| pH 8,5; 4,1% Ethanol 0,28 µmol/l rFIXa | 100 | 96,2 | 86,2 | 77,5 | 63,8 | 48,8 |
| pH 7,4; 33% Ethylenglykol 0,057 µmol/l rFIXa | 100 | 100 | 85,4 | 62,6 | 41,0 | 28,9 |
| pH 8,5; 33% Ethylenglykol 0,028 µmol/l rFIXa | 100 | 97,9 | 88,4 | 78,4 | 66,0 | 45,4 |

### Ergebnis:

Die Hemmung von rFIXa durch einen synthetischen Inhibitor kann bei unterschiedlichem pH (pH 7-9) und unterschiedlichem Gehalt eines Alkohols (vorzugsweise Ethanol oder Ethylenglykol) untersucht werden. Für eine 50%ige Hemmung von rFIXa wurden für den Hemmstoff TIPPS-(3-Am)Phe-iNip-OH IC₅₀-Werte zwischen 20 und 50 µmol/l, mit dem Hemmstoff βNAPS-(3-Am)Phe-TIC-OH IC₅₀-Werte zwischen 60 und 150 µmol/l gefunden. Bei hoher Ethylenglykol-Konzentration und pH 8,5 ist die Hemmwirkung am empfindlichsten nachzuweisen. Dieser Ansatz ist als Screening-Verfahren zur Suche von FIXa-Hemmstoffen geeignet, da außerordentlich geringe Mengen rFIXa benötigt werden und die hohe Alkoholkonzentrationen die Lösung der zu testenden Substanzen erleichtert.

### Beispiel 17

### Ermittlung der Dissoziationskonstante Kᵢ nach dem Verfahren von DIXON

### Testansatz (Konzentration im Test)

- 42 mmol/l Tris-HCl, pH 8,5
- 82 mmol/l NaCl
- 4,1 mmol/l CaCl₂
- 33% Ethylenglykol
- Inhibitor in abgestuften Konzentrationen (0, 20,4 bzw. 40,8 µmol/l)
- MeSO₂-D-HHT-Gly-Arg-pNA (1,02, 0,51 bzw. 0,25 mmol/l)
- 0,14 µmol/l rFIXa (human)
   bzw.
- MeSO₂-D-HHT-Gly-Arg-pNA (1,02, 0,51 bzw. 0,25 mmol/l)
- 0,07 µmol/l nativer FIXa (human)
   bzw.
- MeSO₂-D-CHG-Gly-Arg-pNA (1,02, 0,51 bzw. 0,25 mmol/l)
- 0,057 µmol/l rFIXa (human)

Als Hemmstoff wurde TIPPS-(3-Am)Phe-iNip-OH eingesetzt und beispielhaft die Hemmwirkung für zwei Substrate bei optimalen Bedingungen (pH 8,5, 33% Ethylenglykol) ermittelt.

**Tabelle 9a**

| **Substrat: MeSO**_{**2**}**-D-HHT-Gly-Arg-pNA, 0,14 µmol/l rFIXa** | | | |
|---|---|---|---|
| | **Inhibitor-Konzentration [µmol/l] 1/V [min/mE]** | | |
| **Substrat-Konzentration** | 0 | 20,4 | 40,8 |
| 1,02 µmol/l | 9,5 | 16,6 | 31,2 |
| 0,51 µmol/l | 20,9 | 43,5 | 90,9 |
| 0,25 µmol/l | 33,2 | 69,4 | 137 |

**Tabelle 9b**

| **Substrat: MeSO**_{**2**}**-D-HHT-Gly-Arg-pNA, 0,07 µmol/l nativer FIXa** | | | |
|---|---|---|---|
| | **Inhibitor-Konzentration [µmol/l] 1/V [min/mE]** | | |
| **Substrat-Konzentration** | 0 | 20,4 | 40,8 |
| 1,02 µmol/l | 16,4 | 29,5 | 53,2 |
| 0,51 µmol/l | 38,6 | 77,5 | 154 |
| 0,25 µmol/l | 59,5 | 129 | 255 |

**Tabelle 9c**

| **Substrat: MeSO**_{**2**}**-D-CHG-Gly-Arg-pNA, 0,057 µmol/l rFIXa** | | | |
|---|---|---|---|
| | **Inhibitor-Konzentration [µmol/l] 1/V (min/mE)** | | |
| **Substrat-Konzentration** | 0 | 20,4 | 40,8 |
| 1,02 µmol/l | 15,5 | 23,8 | 44,6 |
| 0,51 µmol/l | 28,7 | 55,6 | 109 |
| 0,25 µmol/l | 43,1 | 78,1 | 153 |

### Ergebnis:

Die Bestimmung der Dissoziationskonstante Kᵢ nach DIXON für die Hemmung von rekombinanten bzw. nativen humanen FIXa durch TIPPS-(3-Am)Phe-iNip-OH wurde bei pH 8,5 und optimaler Ethylenglykol-Konzentration (33%) durchgeführt. Für die Hemmung des rekombinanten bzw. nativen FIXa wurden unter Verwendung der in den Tabellen 9a und 9b zusammengefaßten Werte graphisch vergleichbare Kᵢ-Werte bestimmt (12 bzw. 9 µmol/l). Auch bei Einsatz der Substrate MeSO₂-D-HHT-Gly-Arg-pNA und MeSO₂-D-CHG-Gly-Arg-pNA ergeben sich für die Hemmung von rFIXa vergleichbare Kᵢ-Werte (12 bzw 16 µmol/l). Der gewählte Ansatz mit hoher Ethylenglykol-Konzentration eignet sich insbesondere für die Bestimmung von Hemmkonstanten, da geringe rFIXa-Konzentrationen benötigt werden. Hohe Alkoholkonzentrationen erleichtern zusätzlich die Lösung der zu testenden Substanzen.

### Referenzliste

**Bang, N.U.**, et al, EP 0 191 606
**Bergmeyer, H.U**. (ed.): Methods of Enzymatic Analysis, Vol. V, chapter 3, 3rd ed., Academic Press, New York (1983)
**Bharadwaj, D**., et al., J. Biol. Chem. 270, 6537-6542 (1995)
**Blow, D.M.**, Acc. Chem. Res. 9, 145-152 (1976)
**Brinkmann, U.**, et al., Gene 85, 109114 (1989)
**Davie, E.W**., et al., Biochem. 30, 10363-10379 (1991)
**Esmon, C.T.**, Prothrombin activation, doctoral dissertation, Washington University, St. Louis, MO (1973)
**Europäische Patentanmeldung Nr. 96 109 288.9**
**Europäische Patentanmeldung Nr. 96 110 959.2**
**Europäisches Patent EP-B 0 034 122**
**Fujikawa, K**., et al., Biochem. 11, 4892-4898 (1972)
**Furie, B.**; Furie, B.C., Cell 53, 505- 518 (1988)
**Grodberg, J**.; Dunn, J.J., J. Bacteriol. 170, 1245-1253 (1988)
**Hagen, F.S**., et al, EP 0 200 421
**Hertzberg, M.S.**, et al., J. Biol. Chem. 267, 14759-14766 (1992)
**Holly, R.D**.; Foster, D.C., WO 93/13208
**Hopfner, K.-P.**; Kopetzki, E., Europäische Patentanmeldung Nr. 96 110 959.2
**Kopetzki, E.**, et al., WO 93/09144
**Laemmli, U.K**., Nature 227, 680-685 (1970)
**Lin, S.-W**., et al., J. Biol. Chem. 265, 144-150 (1990)
**McGraw, R.A**., et al., Proc. Natl. Acad. Sci. USA 82, 2847-2851 (1985)
**Medved, L.V.**, et al., J. Biol. Chem. 270, 13652-13659 (1995)
**Nicolaisen, E.M**., et al., WO 88/10295
**Pedersen, A.H**., et al., Biochem. 28, 9391-9336 (1989)
**Polgar, L**., : Structure and function of serine proteases. In: Mechanisms of protein action. Boca Raton, Florida, CRC Press, chapter 3 (1989)
**Rezaie, A.R**, et al., J. Biol. Chem. 268, 8176-8180 (1993)
**Rezaie, A.R.**; Esmon, C.T., J. Biol. Chem. 269; 21495-21499 (1994)
**Sambrook, J.**; Fritsch, E.F.; Maniatis,T.: Molecular cloning: A laboratory manual. Cold Spring Harbor Press, Cold Spring Harbor, New York, (1989)
**Sheehan, J.P.**, et al., J. Biol. Chem. 268, 3639-3645 (1993)
**Svendsen, L.G**., Europäisches Patent EP-B 0 034 122
**Thogersen, H.C.**, et al., WO 94/18227
**Stürzebecher,** et al., J. Enzym. Inhibition 9 (1995) 87
**Stürzebecher,** et al., WO 92/08709, 1991
**Van Dam-Mieras, M.C.E.**, et al.: Blood coagulation factors II, V, VII, VIII, IX, X and XI: Determination with synthetic substrates. In: Bergmeyer, H.U. (ed.): Methods of Enzymatic Analysis, Vol. V, Enzymes 3: Peptidases, Proteinases and Their Inhibitors, page 365-394, 3rd ed., Academic Press, New York (1983)
**Wolf, D.L.**, et al., J. Biol. Chem. 266, 13726-13730 (1991)
**Yee, J.**, et al., J. Biol. Chem. 269, 17965-17970 (1994)
**Zhong, D.G**., et al., Proc. Natl. Acad. Sci. USA 91, 3574-3578 (1994)

## Patentansprüche

1. Verfahren zur Bestimmung von Faktor IXa in einer Probenlösung, welche Faktor IXa enthält oder in welcher Faktor IXa entsteht oder verbraucht wird, dadurch gekennzeichnet, daß der genannten Probenlösung ein bestimmbares Faktor IXa-Substrat und ein mit Wasser einphasig mischbarer Alkohol zugesetzt wird und die katalytische Wirkung von Faktor IXa auf das genannte Substrat über die Spaltung des Faktor IXa-Substrats als Maß für die Faktor IXa-Aktivität bestimmt wird.

2. Verfahren nach Anspruch 1, dadurch gekennzeichnet, daß als Substrat ein Tripeptid mit einer hydrophoben D-Aminosäure am N-Terminus verwendet wird.

3. Verfahren nach Anspruch 2, dadurch gekennzeichnet, daß eine Cyclohexyl-substituierte hydrophobe D-Aminosäure verwendet wird.

4. Verfahren zur Identifikation einer Substanz, welche die Aktivität von Faktor IXa moduliert, dadurch gekennzeichnet, daß
a) ein Faktor IXa-Substrat durch ein Polypeptid mit Faktor IXa-Aktivität in definierter Konzentration in Gegenwart eines mit Wasser einphasig mischbaren Alkohols gespalten wird und die Geschwindigkeit der Spaltung des genannten Substrats als Maß für die Faktor IXa-Aktivität bestimmt wird,
b) die genannte Aktivität in Gegenwart einer Testsubstanz gemessen wird,
c) die Aktivität mit und ohne Testsubstanz verglichen wird und
d) die Differenz der Aktivität als Maß für die Aktivitätsmodulation durch die Testsubstanz verwendet wird.

5. Verfahren nach den Ansprüchen 1 bis 4, dadurch gekennzeichnet, daß als einphasig mit Wasser mischbarer Alkohol Methanol, Ethanol, n- oder i-Propanol, t-Butanol, Glycerin oder insbesondere Ethylenglykol verwendet wird.

6. Reagenz zur Bestimmung von Faktor IXa in einer Probenlösung, welches ein durch Faktor IXa spaltbares chromogenes Substrat, einen einphasig mit Wasser mischbaren Alkohol sowie einen Puffer im Bereich zwischen pH 7 und 10 enthält.

## Claims

1. A method for the determination of factor IXa in a sample solution which contains factor IXa or in which factor IXa is formed or consumed, characterized by adding a measurable factor IXa substrate and an alcohol which is miscible with water to form a single phase to the said sample solution and determining the catalytic effect of factor IXa on the said substrate by means of the cleavage of the factor IXa substrate as a measure for the factor IXa activity.

2. A method according to claim 1, characterized in that a tripeptide with a hydrophobic D-amino acid at the N-terminus is used as the substrate.

3. A method according to claim 2, characterized in that a cyclohexyl-substituted hydrophobic D-amino acid is used.

4. A method for the identification of a substance which modulates the activity of factor IXa, characterized by
a) cleaving a factor IXa substrate by a polypeptide with factor IXa activity in a defined concentration in the presence of an alcohol which is miscible with water to form a single phase and determining the rate of cleavage of the said substrate as a measure for the factor IXa activity,
b) measuring the said activity in the presence of a test substance,
c) comparing the activity with and without the test substance and
d) using the difference of the activity as a measure for the activity modulation by the test substance.

5. A method according to claims 1 to 4, characterized in that methanol, ethanol, n- or i-propanol, t-butanol, glycerol or especially ethylene glycol is used as the alcohol which is miscible with water to form a single phase.

6. A reagent for the determination of factor IXa in a sample solution, which contains a chromogenic substrate cleavable by factor IXa, an alcohol which is miscible with water to form a single phase and a buffer in the range between pH 7 and 10.

## Revendications

1. Procédé de détermination du facteur IXa dans une solution d'échantillon qui contient du facteur IXa ou dans laquelle du facteur IXa est produit ou consommé, caractérisé en ce que l'on ajoute à ladite solution d'échantillon un substrat du facteur IXa pouvant être déterminé et un alcool miscible à l'eau en une seule phase et on détermine l'effet catalytique du facteur IXa sur ledit substrat par l'intermédiaire de la dissociation du substrat de facteur IXa en tant que mesure de l'activité du facteur IXa.

2. Procédé selon la revendication 1, caractérisé en ce que l'on utilise comme substrat un tripeptide possédant un D-aminoacide hydrophobe au niveau de la terminaison N.

3. Procédé selon la revendication 2, caractérisé en ce que l'on utilise un D-aminoacide hydrophobe substitué par un cyclohexyle.

4. Procédé d'identification d'une substance qui module l'activité du facteur IXa, caractérisé en ce que
a) on dissocie un substrat du facteur IXa au moyen d'un polypeptide possédant une activité de facteur IXa en une concentration définie en présence d'un alcool miscible à l'eau en une seule phase et on détermine la vitesse de la dissociation dudit substrat en tant que mesure de l'activité du facteur IXa,
b) on mesure ladite activité en présence d'une substance de test,
c) on compare l'activité avec et sans substance de test, et
d) on utilise la différence d'activité comme mesure de la modulation de l'activité par la substance de test.

5. Procédé selon les revendications 1 à 4, caractérisé en ce que, comme alcool miscible à l'eau en une seule phase, on utilise le méthanol, l'éthanol, le n-propanol ou i-propanol, le t-butanol, le glycérol ou en particulier l'éthylène glycol.

6. Réactif pour la détermination du facteur IXa dans une solution d'échantillon, lequel réactif contient un substrat chromogène pouvant être dissocié par le facteur IXa, un alcool miscible à l'eau en une seule phase ainsi qu'un tampon dans la plage de pH 7 à pH 10.


### SEQUENZPROTOKOLL

(1) ALLGEMEINE ANGABEN:
   (i) ANMELDER:
      (A) NAME: BOEHRINGER MANNHEIM GMBH
      (B) STRASSE: Sandhofer Str. 116
      (C) ORT: Mannheim
      (E) LAND: Germany
      (F) POSTLEITZAHL: D-68305
      (G) TELEFON: 08856/60-3446
      (H) TELEFAX: 08856/60-3451
   (ii) BEZEICHNUNG DER ERFINDUNG: Verfahren zur Bestimmung der katalytischen Aktivitaet von Faktor IXa
   (iii) ANZAHL DER SEQUENZEN: 4
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30B (EPA)
(2) ANGABEN ZU SEQ ID NO: 1:
   (i) SEQUENZKENNZEICHEN:
      (A) LÄNGE: 37 Basenpaare
      (B) ART: Nucleotid
      (C) STRANGFORM: Einzelstrang
      (D) TOPOLOGIE: linear
   (ii) ART DES MOLEKÜLS: other nucleic acid
      (A) BESCHREIBUNG: /desc = "Primer"
   (xi) SEQUENZBESCHREIBUNG: SEQ ID NO: 1:
   AAAAAACCAT GGTTGTTGGT GGAGAAGATG CCAAACC                                  37
(2) ANGABEN ZU SEQ ID NO: 2:
   (i) SEQUENZKENNZEICHEN:
      (A) LÄNGE: 42 Basenpaare
      (B) ART: Nucleotid
      (C) STRANGFORM: Einzelstrang
      (D) TOPOLOGIE: linear
   (ii) ART DES MOLEKÜLS: other nucleic acid
      (A) BESCHREIBUNG: /desc = "Primer"
   (xi) SEQUENZBESCHREIBUNG: SEQ ID NO: 2:
   AAAAAAAAGC TTCATTAAGT GAGCTTTGTT TTTTCCTTAA TC                      42
(2) ANGABEN ZU SEQ ID NO: 3:
   (i) SEQUENZKENNZEICHEN:
      (A) LÄNGE: 39 Basenpaare
      (B) ART: Nucleotid
      (C) STRANGFORM: Einzelstrang
      (D) TOPOLOGIE: linear
   (ii) ART DES MOLEKÜLS: other nucleic acid
      (A) BESCHREIBUNG: /desc = "Primer"
   (xi) SEQUENZBESCHREIBUNG: SEQ ID NO: 3:
   AAAAAACCAT GGATGTAACA TGTAACATTA AGAATGGCA                          39
(2) ANGABEN ZU SEQ ID NO: 4:
   (i) SEQUENZKENNZEICHEN:
      (A) LÄNGE: 24 Basenpaare
      (B) ART: Nucleotid
      (C) STRANGFORM: Einzelstrang
      (D) TOPOLOGIE: linear
   (ii) ART DES MOLEKÜLS: other nucleic acid
      (A) BESCHREIBUNG: /desc = "Primer"
   (xi) SEQUENZBESCHREIBUNG: SEQ ID NO: 4:
   GGGTTCGTCC AGTTCCAGAA GGGC                                          24